# EUROPEAN PATENT SPECIFICATION

(11) **EP 4 146 336 B1**
(45) Date of publication and mention of the grant of the patent: **26.11.2025**
(21) Application number: 21728987.5
(22) Date of filing: 07.05.2021
(51) Int. Cl.: A61N 5/10, A61N 1/06, A61B 18/00, A61N 1/04, A61N 1/05

(54) **SYSTEM FOR TREATING A TUMOUR**
SYSTEM ZUR BEHANDLUNG EINES TUMORS
SYSTÈME POUR TRAITER UNE TUMEUR

(30) Priority: 08.05.2020 IT 202000010387
(43) Date of publication of application: 15.03.2023
(73) Proprietor: Agenzia Nazionale Per Le Nuove Tecnologie, L'Energia E Lo Sviluppo Economico Sostenibile (ENEA), 00196 Roma (IT)
(72) Inventor: PINTO, Rosanna, 01027 Montefiascone (Treviso) (IT); MARINO, Carmela, 00196 Roma (IT); TANORI, Mirella, 00196 Roma (IT); MANCUSO, Mariateresa, 00196 Roma (IT); CASCIATI, Arianna, 00196 Roma (IT); MERLA, Caterina, 00196 Roma (IT); ZAMBOTTI, Alessandro, 00196 Roma (IT)
(74) Representative: Conti, Marco
(86) International application number: PCT/IB2021/053895
(87) International publication number: WO 2021/224876

(56) References cited:
- WO-A1-2020/061192
- WO-A1-98/14238
- WO-A2-2013/096584
- MICHAEL B. SANO ET AL: "Treatment of Cancer In Vitro Using Radiation and High-Frequency Bursts of Submicrosecond Electrical Pulses", IEEE TRANSACTIONS ON BIOMEDICAL ENGINEERING, vol. 65, no. 4, 1 April 2018 (2018-04-01), PISCATAWAY, NJ, USA., pages 928 - 935, XP055760157, ISSN: 0018-9294, DOI: 10.1109/TBME.2017.2734887

## Description

### Technical field

This invention relates to a system for treating a tumour and to a computer program.

The field of this invention is that of medical systems or devices for treating tumours.

### Background art

Known in this field are systems that use ionizing radiations, such as, for example, the systems described in patent documents US5527352A and US5718246A. Radiotherapy can, however, have serious side effects on account of the high radiation doses; for example, if used to treat brain tumours, it may cause serious cognitive impairments.

Also known are systems that use ultrashort electric pulses. More specifically, in some known systems for implementing a therapy known as electrochemotherapy (ECT), tumour cells are treated with pulsed electric fields in combination with chemotherapy drugs to destroy the tumour. Also known are systems for implementing a therapy known as irreversible electroporation (IRE) in which the tumour is destroyed by the high doses of electromagnetic energy applied to the tissues. Examples of these systems or methods for treating a tumour with ultrashort electric pulses are described in the following patent documents: US8512334B2, US9943684B2, US20190299019A1, WO2014/197240A2, WO2015/175570.

In addition, the application of a pulsed electric field in combination with simultaneous irradiation with ionizing radiation is known from patent document RO118847B1.

A widespread need in this technical field is that of having treatment systems and methods that offer the maximum effectiveness in killing the tumour cells (and inhibiting their cell division) and that can, at the same time, safeguard healthy cells and preserve the surrounding tissues as much as possible. This need is felt particularly strongly in the field of the treatment of cancer stem cells (CSC), which is a restricted cell population present in most tumours and which constitutes a source for cancer maintenance and progression.

Furthermore, patent document WO2020/061192A discloses a medical diagnostic system comprising an electrical pulse generator; in this document, after the treatment with electrical impulses, after days or months, a chemotherapy or radiotherapy treatment is applied. The WO2020/061192A system is configured for the treatment of a generic tumor and activation of the immune system. This system has several disadvantages. First of all, it makes it necessary for the patient to undergo several sessions (at least one session for the administration of electrical impulses and one session for chemotherapy or radiotherapy), thus making the treatment long and complex. Furthermore, it is completely silent and does not provide any indication regarding a possible reduction in the radiation dose and related side effects.

### Disclosure of the invention

This invention has for an aim to provide a system and a computer program to overcome the above mentioned disadvantages of the prior art.

This aim is fully achieved by the system and computer program of this disclosure as characterized in the appended claims. The invention is defined by the claims. In the following all aspects, embodiments and examples concerning a method of treatment, in particular methods of treating a tumour, are not claimed and are disclosed for illustrative purposes only.

According to an aspect of it, this disclosure relates to a system for treating a tumour. In particular, the system object of the present description is configured for the treatment of tumor stem cells. The system comprises a pulsed electric field generator, configured to emit electric pulses (specifically ultrashort electric pulses) directed at a zone of a human body to be treated. Preferably, the system also comprises an ionizing radiation generator, configured to emit ionizing rays directed at the zone of the human body to be treated. Thus, the system of this disclosure allows treating tumours with a combination of (ultrashort) electric pulses and ionizing radiation.

The system comprises a timer (or counter, or chronometer or clock) configured to count the time.

The system comprises a control unit. The control unit is connected to the pulsed electric field generator; preferably, the control unit is also connected to the timer. In an embodiment, the control unit is connected to the ionizing radiation generator.

The control unit is programmed to activate the pulsed electric field generator to emit an electric pulse train. After the electric pulse train has been emitted, the control unit is programmed to stop the pulsed electric field generator and to start the timer for counting a predetermined length of time. After the predetermined length of time has elapsed, the control unit is programmed to provide an information item for activating the ionizing radiation generator.

In an embodiment, the information item for activating the ionizing radiation generator may be communicated to a user to help them manage the system and prompt them to activate the ionizing radiation generator.

In fact, tumor cells (in particular, tumor stem cells) are made more vulnerable to the action of ionizing radiation after said predefined period of time has elapsed from the administration of pulsed electric fields. In other words, the administration of pulsed electric fields makes the ionizing radiation, applied at a distance of said predetermined time, more effective in attacking cancer cells.

In this embodiment, the system comprises an output interface, configured to provide a user with the information item for activating the ionizing radiation generator. The output interface may, for example, include a screen on which the predetermined length of time (for example, 3 hours) is displayed. The output interface might be configured to emit a signal (a light, sound, vibration or other signal) when the predetermined length of time has elapsed, to warn the user that it is time to activate the ionizing radiation generator.

In another embodiment, the information item for activating the ionizing radiation generator may be transmitted to the ionizing radiation generator to instruct it to emit a dose of ionizing radiation. Thus, in this embodiment, the control unit is configured to issue a command to activate the ionizing radiation generator when the predetermined length of time has elapsed.

The system comprises an input interface configured to receive an input information item from a user and a value of the predetermined length of time can be set by the control unit as a function of the input information item. For example, the input information item might itself include the predetermined length of time. According to the invention system comprises a database containing a plurality of records, each of which includes a treatment time associated with a type of tumour. Besides the type of tumour, the record might include information such as the patient's age, the state of progression and propagation of the tumour, previous treatments performed on the same tumour and/or on the same patient. Thus, for each type of tumour, the database might include more than one record, hence more than one recommended treatment time depending on other factors, such as those listed above. In this embodiment, the input information item represents a type of tumour to be treated (and, if necessary, other information such as the patient's age, the state of progression and propagation of the tumour, previous treatments performed on the same tumour and/or on the same patient). The control unit is configured to query the database as a function of the input information item, to retrieve the treatment time (specifically, the input time associated with the type of tumour to be treated and with other factors stored in the database), and is also configured to set the treatment time retrieved from the database as a value for the predetermined length of time. The control unit may also retrieve a plurality of times from the database and calculate (for example, through a weighted average) the value to be set for the predetermined length of time. For example, if the database includes a first record containing a first recommended treatment time for a certain type of tumour in patients of a first chronological age, and a second record containing a second recommended treatment time for the same type of tumour in patients of a second chronological age, the control unit can process these data to generate an output information item representing a third recommended treatment time for the same type of tumour in patients of a third chronological age included between the first chronological age and the second chronological age.

The fact of waiting for a predetermined length of time between the application of the pulsed electric fields and the application of the ionizing radiation is of fundamental importance for the efficacy of the treatment, that is to say, for radiosensitizing the cancer cells so that they can be effectively destroyed by applying the ionizing radiation. In effect, if too little time passes between the application of the pulsed electric fields and the application of the ionizing radiation, the biological response to the pulsed electrical fields is not optimum radiosensitization and the treatment therefore has little effect. Conversely, if too much time passes between the application of the pulsed electric fields and the application of the ionizing radiation, their combined effect is diminished on account of greater cell recovery and, in this case, too, the efficacy of the treatment is lowered. Preferably, the predetermined length of time is between 1 h and 15 h. More specifically, the predetermined length of time for treating cancer stem cells is between 2 h and 6 h or between 2 h and 4 h; preferably, it is 3 h. For other types of tumour, the predetermined length of time might be longer, for example, between 7 and 10 h or between 9 and 12 h. It should be noted that this predetermined period of time is short enough to allow the administration of electrical impulses and radiotherapy in a single session, without the need to operate several separate sessions on the patient.

Preferably, the number of electric pulses of the pulse train is low compared to prior art treatments; for example, the pulse train includes a number of electric pulses between 3 and 10, or between 4 and 7 or between 4 and 6. Preferably, the pulse train includes 5 pulses. It is stressed that the number of pulses cannot be reduced below a minimum threshold (for example, 3 or 4), being the minimum level for the appearance of the biological response; and what is more, to limit the possible thermal effects that can damage the surrounding tissues, this value cannot be increased indiscriminately.

Preferably, each electric pulse of the pulse train has an amplitude (that is, an electric field) of between 0.1 MV/m and 0.5MV/m, and more preferably, between 0.2 MV/m and 0.4MV/m or between 0.25 MV/m and 0.35 MV/m (for example, 0.3 MV/m).

Preferably, the pulse train has an energy per unit volume (or energy density) of between 4 and 70 MJ/m³, or between 10 and 60, or between 20 and 50 (for example, 30 MJ/m³). Preferably, each electric pulse of the pulse train has an energy density of between 1 and 2 MJ/m³ (for example, 1.5 MJ/m³).

It should be noted that the energy, the energy density, that is to say, the energy applied per unit volume (calculated as W=σ×(τ×DC×NI)×E2 is measured in J/m³, where W is the energy density, σ the conductivity of the medium (S/m) to which the electric pulses are applied, τ the period of the signal (s), DC the duty cycle of the signal expressed as a percentage, NI the number of pulses applied and E the electric field applied (V/m).

Each electric pulse may have an exponential shape or a square shape or other shapes. The duration considered for each pulse was 50% of the peak value.

The frequency of the electric pulses of the pulse train is preferably between 0.8 and 1.2 Hz., for example, 1 Hz.

The duration of each electric pulse of the pulse train is preferably between 20 and 60 µs, or between 30 and 50 µs, or between 35 and 45 µs; for example, 40 µs. It should be noted that the duration of the pulses might be even lower, for example in the order of tens of nanoseconds or picoseconds, or longer, for example, in the order of milliseconds.

Preferably, the ionizing rays are X-rays. The dose applied (in a single application) is preferably less than 10 Gy, or less than 8 Gy, or less than 6 Gy, or less than 5 Gy; more specifically, it may be between 1 Gy and 4 Gy, or between 1 Gy and 3 Gy (for example, 2 Gy). This dose is low compared to many prior art systems and therefore has a lower impact on the surrounding tissues.

In fact, the administration of pulsed electric fields makes the subsequent radiotherapy more effective, even with reduced doses of radiation imparted.

This disclosure also provides a method for controlling a system for treating a tumour (preferably, for treating cancer stem cells, specifically medulloblastoma).

It should be noted that cancer stem cells are a subpopulation of cancer cells, usually resistant to radiotherapy and chemotherapy.

The system is a system according to one or more aspects of this disclosure. The method comprises a step of activating the pulsed electric field generator to emit an electric pulse train. After emitting the electric pulse train, the method comprises a step of stopping the pulsed electric field generator. At the same time, the method comprises a step of starting a timer for counting a predetermined length of time. After the predetermined length of time has elapsed, the method comprises a step of providing an information item for activating the ionizing radiation generator. In an embodiment, the information item for activating the ionizing radiation generator is supplied to the user through an output interface; that way, the user knows when to activate the ionizing radiation generator. In an embodiment, the information item for activating the ionizing radiation generator might be a command issued by the control unit to activate the ionizing radiation generator.

In an embodiment, the method comprises a step of receiving an input information item representing a type of tumour to be treated, and a step of querying a database as a function of the input information item to retrieve the treatment time associated with the type of tumour to be treated. The database contains a plurality of records, each of which includes a treatment time associated with a type of tumour. The method then comprises a step of setting the treatment time retrieved from the database as the value for the predetermined length of time.

It should be noted that the electric pulse train is a pulse train according to one or more of the aspects of this disclosure; more specifically, it includes a number of electric pulses between 3 and 10, or between 4 and 7 or between 4 and 6 (preferably, the pulse train includes 5 pulses).

This disclosure also provides a computer program comprising operating instructions configured to perform the steps of the method for controlling a system for treating a tumour according to one or more aspects of this disclosure.

This disclosure also provides a method for treating a tumour. More specifically, the method comprises treating cancer stem cells; still more specifically, the method comprises treating cancer stem cells of a medulloblastoma. The method for treating the tumour (or cancer stem cells) comprises a step of placing a pulsed electric field generator in the proximity of a zone of a human body to be treated, and a step of emitting a train of electric pulses with the pulsed electric field generator (the pulses being pulses according to one or more of the aspects of this disclosure). The method for treating the tumour also includes a step of placing an ionizing radiation generator in the proximity of the zone of the human body to be treated, and a step of emitting a dose of ionizing radiation with the ionizing radiation generator. The step of emitting the ionizing radiation is carried out after the step of emitting the electric pulses. More specifically, the step of emitting the ionizing radiation is carried out when a predetermined length of time from the step of emitting the electric pulse train has elapsed. The predetermined length of time is between 1 h and 15 h, or between 2 h and 6 h, or between 2 h and 4 h; preferably, it is substantially equal to 3 h.

### Brief description of the drawings

These and other features will become more apparent from the following description of a preferred embodiment, illustrated by way of non-limiting example in the accompanying drawings, in which:

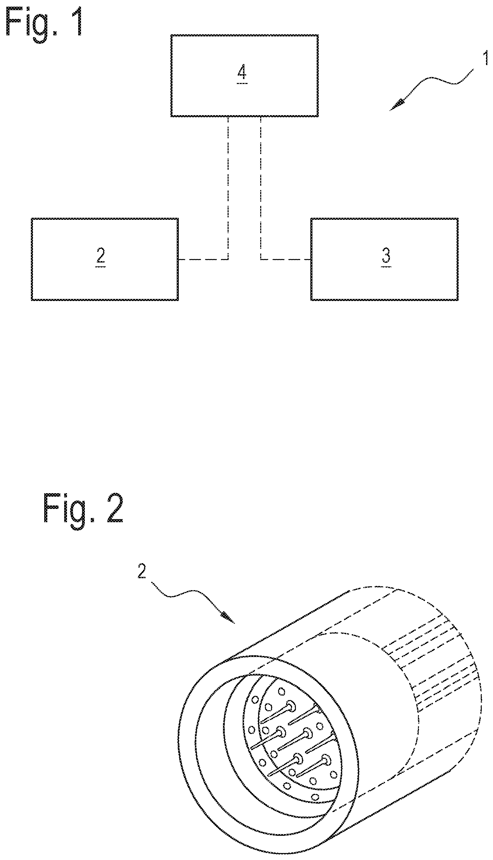

- Figure 1 represents a system for treating a tumour according to one or more aspects of this disclosure;
- Figure 2 shows a pulsed electric field applicator of the system of Figure 1;
- Figures 3-12 show the results of experiments conducted using the system of this disclosure for the treatment of cancer stem cells;
- Figure 13 shows an electric pulse profile of the pulse train emitted by the pulsed electric field generator of Figure 2.

### Detailed description of preferred embodiments of the invention

With reference to the accompanying drawings, the numeral 1 denotes a system for treating a tumour. The system 1 comprises a pulsed electric field generator 2 and an ionizing radiation generator 3. The system 1 comprises a control unit 4. The control unit 4 is configured to control the pulsed electric field generator 2 and, in an embodiment, the ionizing radiation generator 3.

The accompanying drawings show the results of the experimental tests conducted. More specifically, the experimental tests were conducted on a human medulloblastoma cell line (D283 Med ATCC^{®}) characterized by a high percentage (greater than 95%) with stem cell phenotype. Besides these cells, which were considered as an example of medulloblastoma cancer stem cells (CSC), non-tumoral human brain cells, specifically astrocytes (NHA Lonza, Catalog #: CC-2565) were also exposed to the treatment to check the selectivity of the treatment, that is to say, to check the whether the treatment was effective in destroying the tumour cells without affecting the non-tumoral cells.

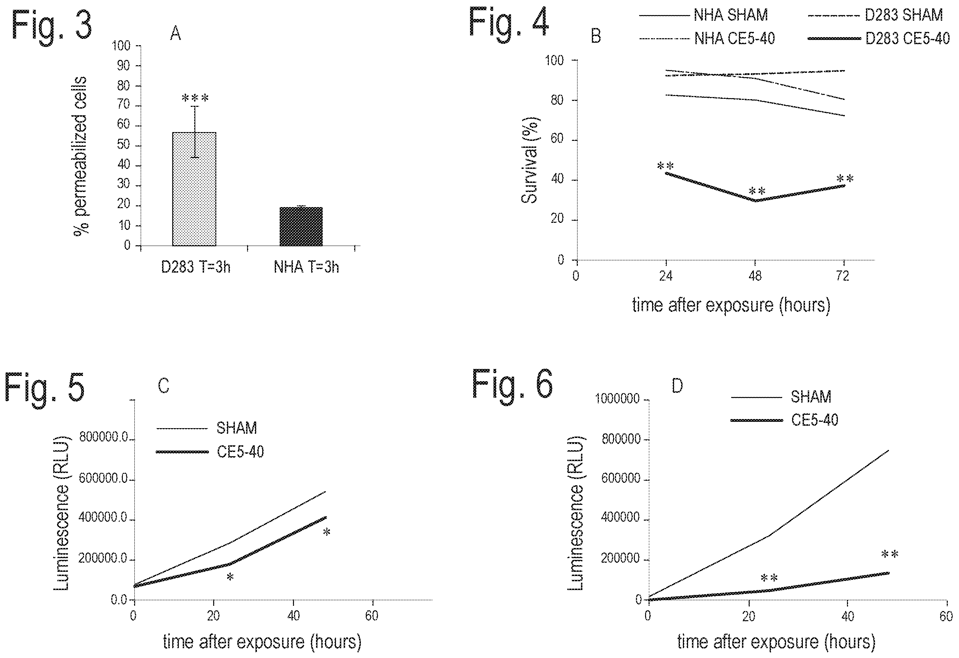

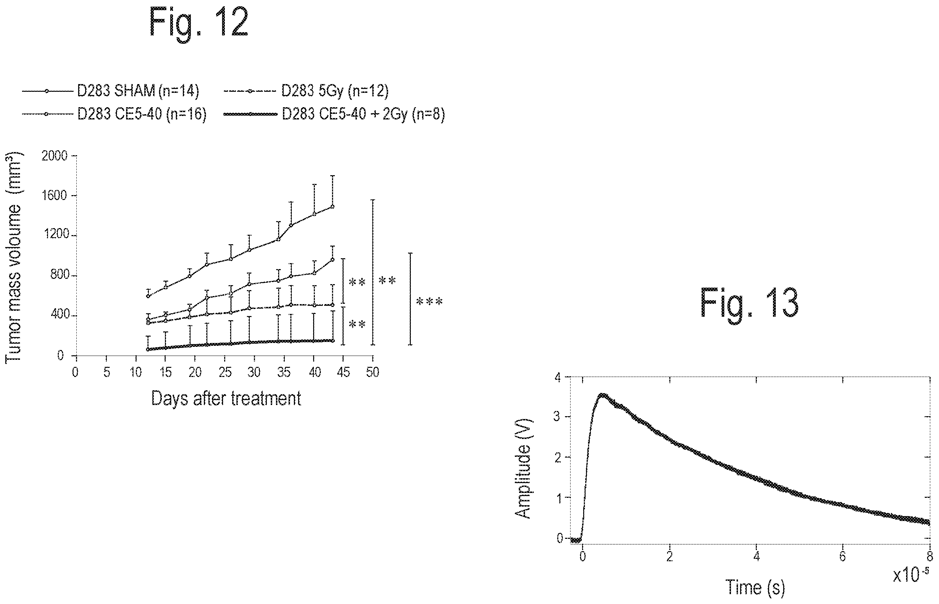

Figures 3-9 show the results of applying a train of 5 electric pulses of exponential shape (that is, the shape illustrated in Figure 13) having an amplitude of 0.3 MV/m and a duration of 40 µs each; this electric pulse train is hereinafter denoted by the label CE5-40.

A first result, represented in Figure 3, showed that approximately 80% of the D283 cells were permeabilized after exposure (after 3 h). In effect, the electric pulses cause the migration of the electric charges in the cancer cells and modify the orientation of the dipolar molecules, causing an increase in the transmembrane potential. This polarization results in structural defects in the plasma membranes, which thus become more permeable. The NHA cells, on the other hand, do not undergo any substantial change in their permeability after 3 hours because it would be necessary to induce a higher transmembrane potential in these cells to produce significant structural defects.

In line with this result, a high cell death rate (assessed with trypan blue) was found in the D283 cells and not in the NHA cells at all the times examined (Figure 4). Furthermore, exposure to CE5-40 induced a statistically significant block in cell proliferation of both cell lines but in the D283 cells with an efficacy 6.9 times higher at 24 hours from exposure and 8.2 times higher after 48 hours (Figure 5) compared to the NHA cells (Figure 6).

These results show that the application of CE5-40 acts selectively on the CSCs, without damaging the healthy cells (NHA).

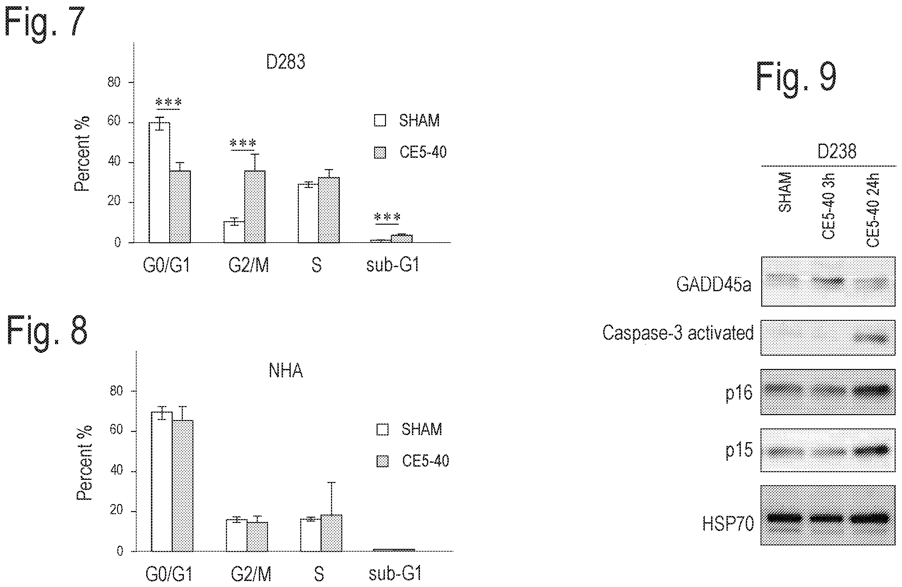

Following these results, the state of the cell cycle was analysed 24 hours after exposure using the cytofluorimetric method (propidium iodide staining). The results revealed, in the group of cells exposed to CE5-40, a significant decrease of the G0/G1 phase of the cell cycle and a significant increase of the Sub-G1 and G2/M phase, compared to the group of cells that was not exposed, denoted as "SHAM"; this suggests the induction of significant cell death levels and G2/M phase arrest in the surviving cells (as shown in Figure 7). No perturbation was, however, revealed in the NHA cell cycle (as shown in Figure 8).

To identify the molecular mechanisms behind this different response, the expression profile of the genes involved in the cell cycle 24 hours after exposure was analysed using the *Human Cell Cycle RT2 Profiler PCR Array* tool. Bioinformatic analysis, namely Signaling Pathway Impact Analysis (SPIA), showed that the GADD45a gene is highly involved in the G2/M arrest and plays a key role in inducing apoptosis and senescence. Moreover, a protein expression analysis (western blot), the results of which are given in Figure 9, which shows the analysis of the proteins in the D283 cells without treatment and after 3 hours and 24 hours from treatment with CE5-40, confirmed that the GADD45a gene mediates the biological processes after caspase-3 and p15 and p16 activation.

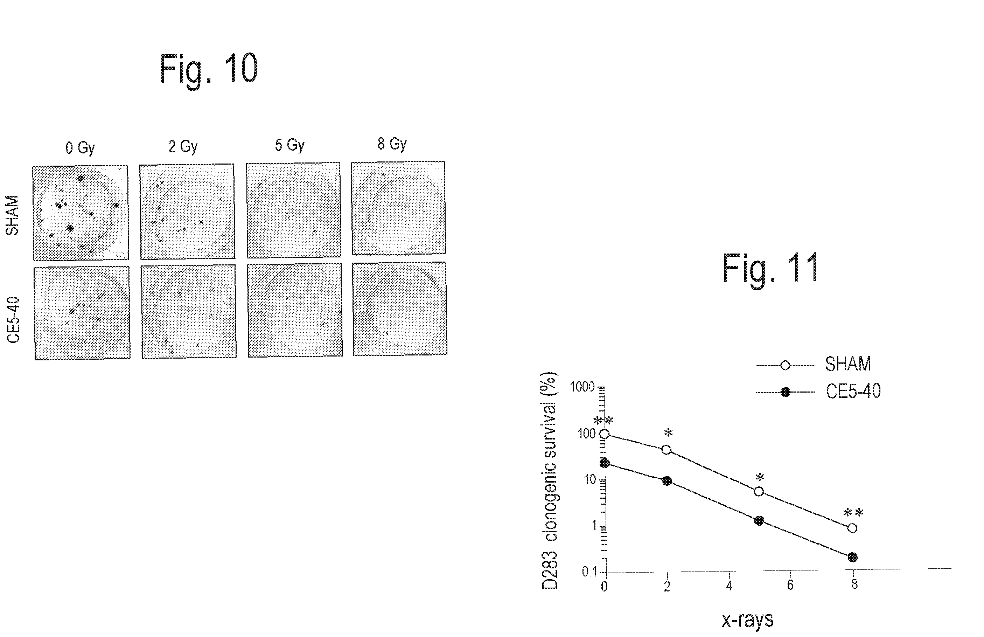

Figures 10-11 show the results of *in vitro* tests on CSCs, involving exposure to ionizing radiation (X-rays in increasing doses of 2, 5 and 8 Gy) following application of CE5-40.

More specifically, Figure 10 shows an image representing the results of a clonogenic test for the D283 line rich in CSCs; Figure 11 shows the reduction of clones following treatment with CE5-40 and exposure to increasing doses of ionizing radiation, compared with the reduction of clones following exposure to the same doses of ionizing radiation of "SHAM" cells, that is, cells not previously exposed to CE5-40. These results show the efficacy of combining the two treatments, namely the pulsed electric field and ionizing radiation treatments. In particular, application of pulsed electric fields allows obtaining a significant reduction of clones even at not too high doses of ionizing radiation. For example, combining CE5-40 with 2 Gy is as efficacious as applying 5 Gy without applying CE5-40. Thus, these tests show that the application of CE5-40 has a radiosensitizing effect on the CSCs.

Figure 12 shows the results of *in vivo* tests on CSCs, involving exposure to ionizing radiation (X-rays in increasing doses of 2, 5 and 8 Gy) following application of CE5-40. For this purpose, the cells pretreated with electric pulses were inoculated into the sides of CD1nu/nu mice and the inoculation site was irradiated 3 hours after inoculation. Tumour growth was monitored for 43 days. More specifically, Figure 12 shows the tumour growth curves in D283 SHAM (untreated) cells, in D283 cells treated with CE5-40 but not with X-rays, in D283 cells treated with a 5 Gy dose of X-rays but not with CE5-40 and, lastly, in D283 cells treated with CE5-40 and with a 2 Gy dose of X-rays. It is noted that the combined action of electric pulses and X-rays in a dose of 2 Gy is more efficacious not only than treatment just with CE5-40 pulses but also than treatment just with X-rays in a dose of 5 Gy. The results of Figure 12 thus show a significant reduction in tumour growth using a combined treatment with CE5-40 followed by a 2 Gy dose of X-rays, preferably applied approximately 3 hours after the electric pulse treatment. This result shows that the electric pulses are capable of acting as radiosensitizing agent by selectively altering the physiology of the CSCs and thus enhancing the response of the CSCs to the X-rays. Thus, the application of electric pulses combined with the subsequent application of ionizing radiation allows obtaining efficacious results even with reduced doses of radiation, hence limiting the side effects.

## Claims

1. A system (1) for treating a tumour, comprising:
- a pulsed electric field generator (2), configured to emit electric pulses directed at a zone of a human body to be treated;
- an ionizing radiation generator (3), configured to emit ionizing rays directed at the zone of the human body to be treated,
wherein the system further comprises
- a timer, configured for counting a length of time;
- a control unit (4), programmed to activate the pulsed electric field generator (2) to emit an electric pulse train and, after emitting the electric pulse train, to stop the pulsed electric field generator (2) and to start the timer to count a predetermined length of time and, after that length of time has elapsed, to provide an information item for activating the ionizing radiation generator in order to activate the ionizing radiation generator (3);
- an input interface configured to receive an input information item from a user, wherein the control unit is programmed to set a value of the predetermined length of time as a function of the input information item;
- a database containing a plurality of records, each of which includes a treatment time associated with a type of tumour, wherein the input information item represents a type of tumour to be treated and wherein the control unit (4) is configured to query the database as a function of the input information item, to retrieve the treatment time, and is also configured to set the treatment time retrieved from the database as a value for the predetermined length of time.

2. The system (1) according to claim 1, comprising an output interface, configured to provide a user with the information item for activating the ionizing radiation generator.

3. The system (1) according to claim 1 or claim 2, wherein the control unit is configured to issue a command to activate the ionizing radiation generator (3) to emit the dose of ionizing rays after the predetermined length of time has elapsed, by means of the information item for activating the ionizing radiation generator.

4. The system (1) according to any one of the preceding claims, wherein the predetermined length of time is between 1 h and 15 h.

5. The system (1) according to any one of the preceding claims, wherein the pulse train includes a number of electric pulses between 4 and 7.

6. The system (1) according to any one of the preceding claims, wherein each electric pulse of the pulse train has an amplitude of between 0.2 MV/m and 0.4MV/m.

7. The system (1) according to any one of the preceding claims, wherein the pulse train has an energy density of between 4 and 70 MJ/m³.

8. The system (1) according to any one of the preceding claims, wherein each electric pulse of the pulse train has an energy density of between 1 and 2 MJ/m³.

9. The system (1) according to any one of the preceding claims, wherein the ionizing rays are X-rays and the dose is between 1 Gy and 4 Gy.

10. A computer program comprising operating instructions configured to perform the following steps, when run on a computer:
- activating a pulsed electric field generator (2) to emit an electric pulse train,
- after emitting the electric pulse train, stopping the pulsed electric field generator (2);
- starting a timer for counting a predetermined length of time from the stop of the pulsed electric field generator (2),
- after the predetermined length of time has elapsed, providing an information item for activating a ionizing radiation generator, to activate the ionizing radiation generator (3);
- receiving an input information item representing a type of tumour to be treated;
- querying a database as a function of the input information item, wherein the database contains a plurality of records, each of which includes a treatment time associated with a type of tumour, in order to retrieve the treatment time associated with the type of tumour to be treated;
- setting the treatment time retrieved from the database as the value for the predetermined length of time.

## Patentansprüche

1. System (1) zur Behandlung eines Tumors, umfassend:
- einen Generator für ein gepulstes elektrisches Feld (2), der ausgelegt ist, um elektrische Impulse zu emittieren, die auf eine Zone eines menschlichen Körpers gerichtet sind, die behandelt werden soll;
- einen Generator für ionisierende Strahlung (3), der so ausgelegt ist, dass er ionisierende Strahlen emittiert, die auf die Zone des menschlichen Körpers gerichtet sind, die behandelt werden soll,
wobei das System weiter umfasst
- einen Timer, der zum Zählen einer Zeitdauer ausgelegt ist;
- eine Steuereinheit (4), die programmiert ist, um den Generator für ein gepulstes elektrisches Feld (2) zu aktivieren, um eine elektrische Impulsfolge zu emittieren und nach dem Emittieren der elektrischen Impulsfolge den Generator für ein gepulstes elektrisches Feld (2) zu stoppen und den Timer zu starten, um eine vorbestimmte Zeitdauer zu zählen und nach Ablauf dieser Zeitdauer eine Information zum Aktivieren des Generators für ionisierende Strahlung bereitzustellen, um den Generator für ionisierende Strahlung (3) zu aktivieren;
- eine Eingabeschnittstelle, die ausgelegt ist, um eine Eingabeinformation von einem Benutzer zu empfangen, wobei die Steuereinheit programmiert ist, um einen Wert der vorbestimmten Zeitdauer als eine Funktion der Eingabeinformation einzustellen;
- eine Datenbank, die eine Vielzahl von Datensätzen enthält, von denen ein jeder eine Behandlungszeit einschließt, die mit einer Art von Tumor assoziiert ist, wobei die Eingabeinformation eine Art von Tumor darstellt, der behandelt werden soll, und wobei die Steuereinheit (4) ausgelegt ist, um die Datenbank als eine Funktion der Eingabeinformation abzufragen, um die Behandlungszeit abzurufen, und ist auch ausgelegt, um die Behandlungszeit, die aus der Datenbank abgerufen wird, als einen Wert für die vorbestimmte Zeitdauer einzustellen.

2. System (1) nach Anspruch 1, umfassend eine Ausgabeschnittstelle, die dazu ausgelegt ist, einem Benutzer die Information zum Aktivieren des Generators für ionisierende Strahlung bereitzustellen.

3. System (1) nach Anspruch 1 oder Anspruch 2, wobei die Steuereinheit ausgelegt ist, um einen Befehl zum Aktivieren des Generators für ionisierende Strahlung (3) zu emittieren, um die Dosis von ionisierenden Strahlen nach Ablauf der vorbestimmten Zeitdauer mittels der Information zum Aktivieren des Generators für ionisierende Strahlung zu emittieren.

4. System (1) nach einem der vorhergehenden Ansprüche, wobei die vorbestimmte Zeitdauer zwischen 1 h und 15 h liegt.

5. System (1) nach einem der vorhergehenden Ansprüche, wobei die Impulsfolge eine Anzahl von elektrischen Impulsen zwischen 4 und 7 einschließt.

6. System (1) nach einem der vorhergehenden Ansprüche, wobei ein jeder elektrische Impuls der Impulsfolge eine Amplitude zwischen 0,2 MV/m und 0,4 MV/m aufweist.

7. System (1) nach einem der vorhergehenden Ansprüche, wobei die Impulsfolge eine Energiedichte zwischen 4 und 70 MJ/m³ aufweist.

8. System (1) nach einem der vorhergehenden Ansprüche, wobei ein jeder elektrische Impuls der Impulsfolge eine Energiedichte zwischen 1 und 2 MJ/m³.

9. System (1) nach einem der vorhergehenden Ansprüche, wobei die ionisierenden Strahlen Röntgenstrahlen sind und die Dosis zwischen 1 Gy und 4 Gy liegt.

10. Computerprogramm, das Betriebsanweisungen umfasst, die ausgelegt sind, um die folgenden Schritte auszuführen, wenn sie auf einem Computer ausgeführt werden:
- Aktivieren eines Generators für ein gepulstes elektrisches Feld (2), um eine elektrische Impulsfolge zu emittieren,
- nach dem Emittieren der elektrischen Impulsfolge, Stoppen des Generators für ein gepulstes elektrisches Feld (2);
- Starten eines Timers zum Zählen einer vorbestimmten Zeitdauer ab dem Stopp des Generators für ein gepulstes elektrisches Feld (2),
- nach Ablauf der vorbestimmten Zeitdauer, Bereitstellen einer Information zum Aktivieren eines Generators für ionisierende Strahlung, um den Generator für ionisierende Strahlung (3) zu aktivieren;
- Empfangen einer Eingabeinformation, die eine Art von Tumor darstellt, der behandelt werden soll;
- Abfragen einer Datenbank als Funktion der Eingabeinformation, wobei die Datenbank eine Vielzahl von Datensätzen enthält, von denen ein jeder eine Behandlungszeit einschließt, die mit einer Art von Tumor assoziiert ist, um die Behandlungszeit abzurufen, die mit der Art von Tumor assoziiert ist, der behandelt werden soll;
- Einstellen der aus der Datenbank abgerufenen Behandlungszeit als den Wert für die vorbestimmte Zeitdauer.

## Revendications

1. Système (1) pour traiter une tumeur, comprenant :
- un générateur de champ électrique pulsé (2), configuré pour émettre des impulsions électriques dirigées en correspondance d'une zone d'un corps humain à traiter ;
- un générateur de rayonnement ionisant (3), configuré pour émettre des rayons ionisants dirigés en correspondance de la zone du corps humain à traiter,
dans lequel le système comprend en outre
- une minuterie, configurée pour compter une durée ;
- une unité de commande (4), programmée pour activer le générateur de champ électrique pulsé (2) afin d'émettre un train d'impulsions électriques et, après avoir émis le train d'impulsions électriques, pour arrêter le générateur de champ électrique pulsé (2) et pour démarrer la minuterie afin de compter une durée prédéterminée et, une fois cette durée écoulée, pour fournir un élément d'information permettant d'activer le générateur de rayonnement ionisant afin d'activer le générateur de rayonnement ionisant (3) ;
- une interface d'entrée configurée pour recevoir un élément d'information d'entrée provenant d'un utilisateur, dans lequel l'unité de commande est programmée pour définir une valeur de la durée prédéterminée en fonction de l'élément d'information d'entrée ;
- une base de données contenant une pluralité d'enregistrements, chacun d'entre eux incluant une durée de traitement associée à un type de tumeur, dans lequel l'élément d'information d'entrée représente un type de tumeur à traiter et dans lequel l'unité de commande (4) est configurée pour interroger la base de données en fonction de l'élément d'information d'entrée, afin de récupérer la durée de traitement, et est également configurée pour définir la durée de traitement récupérée à partir de la base de données en tant que valeur pour la durée prédéterminée.

2. Système (1) selon la revendication 1, comprenant une interface de sortie, configurée pour fournir à un utilisateur l'élément d'information permettant d'activer le générateur de rayonnement ionisant.

3. Système (1) selon la revendication 1 ou la revendication 2, dans lequel l'unité de commande est configurée pour émettre une commande afin d'activer le générateur de rayonnement ionisant (3) pour émettre la dose de rayons ionisants après que la durée prédéterminée s'est écoulée, au moyen de l'élément d'information pour activer le générateur de rayonnement ionisant.

4. Système (1) selon l'une quelconque des revendications précédentes, dans lequel la durée prédéterminée est comprise entre 1 et 15 heures.

5. Système (1) selon l'une quelconque des revendications précédentes, dans lequel le train d'impulsions inclut un nombre d'impulsions électriques compris entre 4 et 7.

6. Système (1) selon l'une quelconque des revendications précédentes, dans lequel chaque impulsion électrique du train d'impulsions a une amplitude comprise entre 0,2 et 0,4 MV/m.

7. Système (1) selon l'une quelconque des revendications précédentes, dans lequel le train d'impulsions a une densité d'énergie comprise entre 4 et 70 MJ/m³.

8. Système (1) selon l'une quelconque des revendications précédentes, dans lequel chaque impulsion électrique du train d'impulsions a une densité d'énergie comprise entre 1 et 2 MJ/m³.

9. Système (1) selon l'une quelconque des revendications précédentes, dans lequel les rayons ionisants sont des rayons X et la dose est comprise entre 1 et 4 Gy.

10. Programme informatique comprenant des instructions d'exécution configurées pour effectuer les étapes suivantes, lorsqu'il est exécuté sur un ordinateur :
- activer un générateur de champ électrique pulsé (2) pour émettre un train d'impulsions électriques,
- après avoir émis le train d'impulsions électriques, arrêter le générateur de champ électrique pulsé (2) ;
- démarrer une minuterie pour compter une durée prédéterminée à partir de l'arrêt du générateur de champ électrique pulsé (2),
- après que la durée prédéterminée s'est écoulée, fournir un élément d'information pour activer un générateur de rayonnement ionisant, afin d'activer le générateur de rayonnement ionisant (3) ;
- recevoir un élément d'information d'entrée représentant un type de tumeur à traiter ;
- interroger une base de données en fonction de l'élément d'information d'entrée, dans lequel la base de données contient une pluralité d'enregistrements, chacun d'entre eux incluant une durée de traitement associée à un type de tumeur, afin de récupérer la durée de traitement associée au type de tumeur à traiter ;
- définir la durée de traitement récupérée à partir de la base de données comme étant la valeur pour la durée prédéterminée.
